# EUROPÄISCHE PATENTANMELDUNG

(11) **EP 2 108 375 A1**
(43) Veröffentlichungstag der Anmeldung: **14.10.2009**
(21) Anmeldenummer: 08007133.5
(22) Anmeldetag: 10.04.2008
(51) Int. Cl.: A61K 39/265, C12N 15/00

(54) **Lebendimpfstoffzusammensetzung**

(71) Anmelder: RIEMSER Arzneimittel AG, 17493 Greifswald - Insel Riems (DE)
(72) Erfinder: Kalthoff, Donata Dr., 17498 Weitenhagen (DE); Beer, Martin Dr., 17498 Neuenkirchen (DE); Küster, Heike Dr., 18435 Stralsund (DE)
(74) Vertreter: Katscher Habermann Patentanwälte

(57) **Zusammenfassung**

Eine Lebendimpfstoffzusammensetzung enthält eine gE- und TK-deletierte BHV-1-Mutante in einem geeigneten Träger und ein nicht viruzides Adjuvans. Das nicht viruzide Adjuvans ist ein Polymer, insbesondere ein Copolymer oder BlockCopolymer oder Polygen^{™}. Der Anteil des nicht viruziden Adjuvans beträgt 5 bis 50 % (v/v), vorzugsweise 10 bis 15 % (v/v) beträgt. Die Lebendimpfstoffzusammensetzung wird zur Herstellung einer Arzneimittelformulierung zur Prophylaxe von BHV-1-Infektionen und BHV-1-vermittelten Erkrankungen verwendet.

## Beschreibung

Die vorliegende Erfindung betrifft eine Lebendimpfstoffzusammensetzung, die Verwendung der Lebendimpfstoffzusammensetzung zur Herstellung einer Arzneimittelformulierung zur Prophylaxe von BHV-1-Infektionen und von BHV-1-vermittelten Erkrankungen sowie ein Verfahren zur Herstellung einer gE- und TK-deletierten BHV-1-Mutante und eine mit diesem Verfahren erhältliche BVH-1-Mutante.

Das Bovine Herpesvirus 1 (BHV-1) ist ein weit verbreiteter Erreger in der Rinderpopulation. Es verursacht respiratorische Erkrankungsbilder, Beeinträchtigungen der Milch- und Mastleistung und Störungen der Reproduktionsraten, die häufig auch subklinisch verlaufen. Es ist das kausative Agens der Infektiösen Bovinen Rindertracheitis (IBR) und der Infektiösen Pustulären Vulvovaginitis/Balanoposthitis (IPV/IBP).

Die Bekämpfung von BHV-1-Infektionen beruht derzeit überwiegend auf einer Impfung mit Glycoprotein E-(gE-)deletierten Markerimpfstoffen und dem Nachweis von gE-Antikörpern, um eine zuverlässige Differenzierung infizierter Tiere von geimpften Tieren zu ermöglichen.

gE-deletierte BHV-1-Impfstoffe werden in Form von abgeschwächten Lebendimpfstoffen oder inaktivierten Impfstoffen angeboten, wobei Lebendimpfstoffe in der Regel einen besseren Schutz verleihen.

Ein Nachteil von BHV-1-Lebendimpfstoffen besteht jedoch darin, dass diese bezüglich einer Kontamination mit Fremdviren, wie z.B. dem Virus der bovinen Rinderdiarrhoe (BVDV), weniger sicher als inaktivierte Impfstoffe sind. Das BVDV kann Krankheitsbilder hervorrufen, die denen der IBR ähneln. Es besteht daher ein Bedarf an Lebendimpfstoffen, die im Wesentlichen keine klinischen Krankheitsbilder hervorrufen und sicher zu identifizieren sind.

Darüber hinaus besteht bei der Verwendung von Lebendimpfstoffen das Risiko, dass diese in einem Latenzzustand im Rind verbleiben und reaktiviert werden können. Um dieses Risiko zu vermindern, wurde ein zweites Markerprotein deletiert. M.J. Kaashoek, F.A.C. van Engelenburg, A. Moerman, A.L.J. Gielkens, F.A.M. Rijsewijk und J.T. van Oirschot beschreiben in Veterinary Microbiology 48, (1996), 143-153, eine BHV-1-Mutante, die in den für das Glycoprotein E und die Thymidinkinase (TK) kodierenden Genorten deletiert ist. Mit dieser Mutante konnte eine protektive Immunität gegen eine Erkrankung hervorgerufen werden. Die erreichte Schutzwirkung überstieg aber nicht die, welche mit einem einfach gE-deletierten Impfvirus erreicht wurde.

Demgegenüber besteht die Aufgabe der vorliegenden Erfindung darin, die Prophylaxe von BHV-1-Infektionen bei Tieren, insbesondere Rindern, zu verbessern.

Zur Lösung dieser Aufgabe wird eine Impfstoffzusammensetzung mit den Merkmalen des Anspruchs 1 sowie deren Verwendung nach Anspruch 6, ein Verfahren zur Herstellung einer gE- und TK-deletierten BHV-1-Mutante mit den Schritten des Anspruchs 7 und eine durch dieses Verfahren erhältliche BHV-1-Mutante gemäß Anspruch 8 vorgeschlagen.

Unter dem Begriff "Adjuvans" wird erfindungsgemäß eine Verbindung verstanden, welche die Einleitung einer Immunantwort auf das Antigen fördert.

Unter dem Begriff "nicht viruzid" wird erfindungsgemäß die Eigenschaft eines Adjuvans verstanden, bei Zusatz zu einer mit einem Virus beimpften Zellkultur in einer Konzentration gemäß Herstellerangabe im Wesentlichen vergleichbar hohe Zellkulturtiter wie die virusbeimpfte Zellkultur alleine zu ergeben.

Unter den Bezeichnungen "x-deletierte BHV-1-Mutante" bzw. "BHV-1Δx" wird erfindungsgemäß eine BHV-1-Mutante verstanden, deren für das Protein x kodierender Genort wenigstens teilweise, bevorzugt vollständig deletiert ist mit der Konsequenz, dass die Mutante nicht in der Lage ist, das Protein x zu exprimieren.

Überraschenderweise hat es sich herausgestellt, dass durch Zusatz eines nicht viruziden Adjuvans zu einer Lebendimpfstoffzusammensetzung, die eine gE- und TK-deletierte BHV-1-Mutante in einem geeigneten Träger enthält, die T-Cell-Immunität im geimpften Tier stimuliert wird, sodass auch die Anforderungen an einen viralen Impfstoff für neonatale Patienten erfüllt werden. Darüber hinaus werden durch die erfindungsgemäße Lebendimpfstoffzusammensetzung hohe Antikörpertiter induziert, die zu einer besonders augeprägten protektiven Immunitätslage und damit zu einem umfassenden Schutz vor einer Belastungsinfektion mit virulentem BHV-1 führen. Die erfindungsgemäße gE- und TK-deletierte BHV-1-Mutante erwies sich zudem als vollkommen avirulent und führte bei bei intramuskulärer Immunisierung zu keiner nachweisbaren Replikation des Impfvirus in der nasalen Mukosa. Damit besteht auch die Wahrscheinlichkeit, dass durch das Impfvirus keine Zellen latent infiziert wurden.

Bevorzugt ist das nicht viruzide Adjuvans der erfindungsgemäßen Lebendimpfstoffzusammensetzung ein Polymer, insbesondere ein Copolymer oder Block-Copolymer. Als für die erfindungsgemäße Lebendimpfstoffzusammensetzung besonders geeignet hat sich das von der MVP Laboratories, Inc., Omaha, NE, USA angebotene Adjuvans Polygen^{™} erwiesen.

Als Täger für die gE- und TK-deletierte BHV-1-Mutante eignen sich in der erfindungsgemäßen Lebendimpfstoffzusammensetzung insbesondere permanente Zellinien, wie z.B. Madin-Darby-Bovine-Kidney- (MDBK-) Zelllinien in einem geeigneten Medium. Die Auswahl eines Trägers liegt im Bereich des fachmännischen Könnens.

Die erfindungsgemäße Lebendimpfstoffzusammensetzung enthält das nicht viruzide Adjuvans bevorzugt in einem Anteil von 5 bis 50 % (v/v, beziehungsweise Volumenprozent), besonders bevorzugt in einem Anteil von 10 bis 15 % (v/v).

Die erfindungsgemäße Lebendimpfstoffzusammensetzung kann darüber hinaus weitere Bestandteile enthälten, insbesondere Hilfsstoffe, welche die Haltbarkeit der Lebendimpfstoffzusammensetzung verbessern, z.B. Stabilisatoren für die Lyophilisation.

Typische Impfdosierungen für die gE- und TK-deletierte BHV-1-Mutante in der erfindungsgemäßen Lebendimpfstoffzusammensetzung liegen im Bereich von 10⁵ bis 10⁸ KID₅₀ (Kulturinfektiöse Dosis 50 %).

Die vorliegende Erfindung betrifft auch die Verwendung der erfindungsgemäßen Lebendimpfstoffzusammensetzung zur Herstellung einer Arzneimittelformulierung zur Prophylaxe von BHV-1-Infektionen und BHV-1-vermittelten Erkrankungen.

Zur weiteren Lösung der der Erfindung zugrunde liegenden Aufgabe wird ein Verfahren zur Herstellung einer gE- und TK-deletierten BHV-1-Mutante vorgeschlagen mit den Schritten
A. Transfizieren der DNA von BHV-1ΔgE und ein Rekombinationsplasmid mit einer BAC-Kassette in eukaryotische Zellen.
B. Insertion der BAC-Kassette in die DNA von BHV-1ΔgE zur Bildung von BHV-1ΔgE-BAC-DNA;
C. Elektroporieren von Plasmiden der BHV-1ΔgE-BAC-DNA in elektrokompetente *E*. *coli;*
D. Mutagenisierung des BHV-1ΔgE-BAC-Klons in *E*. *coli* durch homologe Rekombination zur Bildung von BHV-1ΔgEΔTK-BAC-DNA;
E. Selektieren der mutagenisierten BHV-1ΔgEATK-BAC-DNA;
F. Transfizieren der mutagenisierten BHV-1ΔgEΔTK-BAC-DNA in eukaryotische Zellen;
G. Generieren von gE- und TK-deletierten BHV-1-Nachkommen.
sowie die durch dieses Verfahren erhältliche BHV-1-Mutante.

Mit dem erfindungsgemäßen Verfahren ist es gelungen, eine gE- und TK-deletierte BHV-1-Mutante zu konstruieren, die sich *in vitro* nahezu wie ein einzel gE-deletiertes BHV-1 verhielt. So können in Zellkultur einfach hohe Titer erreicht werden und trotz doppelter Attenuierung (gE und TK) werden ausreichende Immunantworten erreicht.

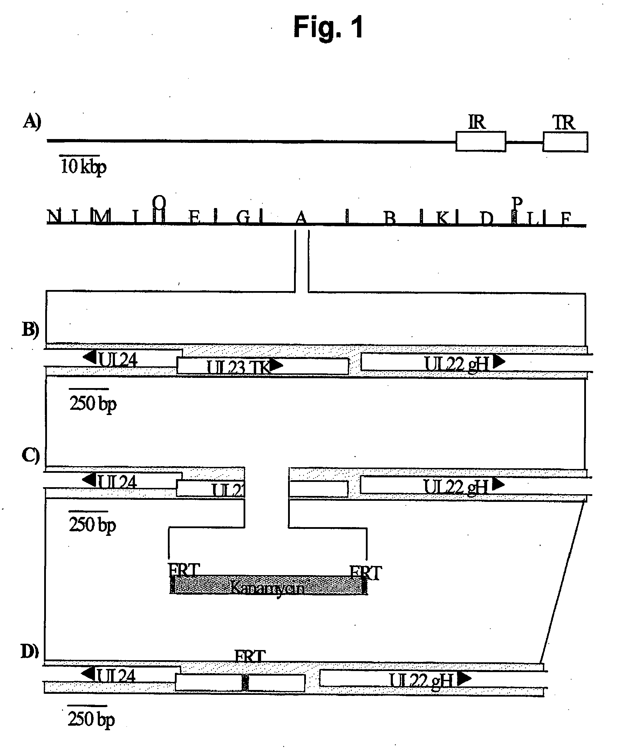

Die vorliegende Erfindung wird im Folgenden anhand eines Beispiels, welches die Erfindung nicht einschränken soll, und unter Bezugnahme auf die angefügten Figuren 1 bis 7 näher erläutert.
Figur 1 zeigt die Konstruktion der ΔTK-Deletionsmutante durch gezielte BAC-Mutagenese: (A) zeigt die Genomorganisation von BHV-1 und die HindIII-Restriktionsfragmentkarte. In (B) ist die Lage des UL23 TK ORFs und seiner benachbarten Leserahmen dargestellt. (C) zeigt die Deletion innerhalb des TK ORF und die Insertion des Kanamycinresistenzgens und (D) den Genomabschnitt nach Elimination des Resistenzgens.

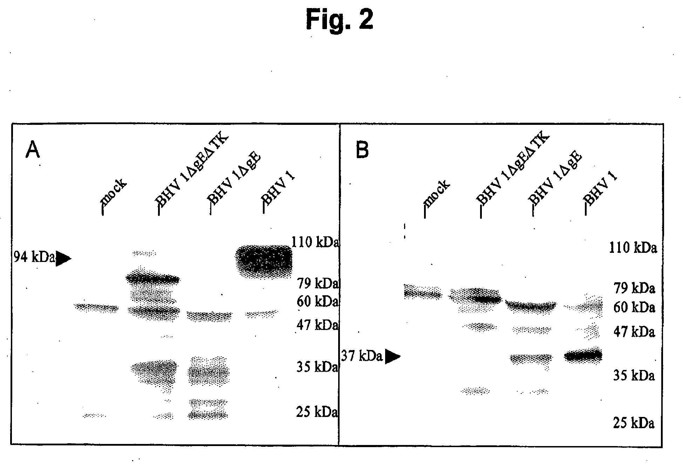

Figur 2 zeigt die Ergebnisse der Western-Blot-Analyse von BHV-1ΔgEΔTK. Die Überprüfung der Proteinexpression von gE und TK erfolgte mittels SDS-(10%)-Polyacrylamid-Elektrophorese und anschließendem Immunoblotting. Rechts neben den Abbildungen ist jeweils ein Größenmarker dargestellt. (A) Der Nachweis einer spezifischen Bande nach Einsatz des monoklonalen Antikörpers gegen gE von ca. 94 kDa (Pfeilspitze) erfolgte nur beim Wildtypvirus (von links nach rechts: uninfizierte Kontrollzellen, BHV-1ΔgEΔTK, BHV-1ΔgE und BHV-1 wt). (B) Zur Untersuchung der Zelllysate wurde der polyklonale Antikörper TK1 eingesetzt. Nur beim Wildtypvirus und bei BHV-11ΔgE gelang die Detektion einer spezifischen Bande von einer ungefähren Größe von 37 kDa (Pfeilspitze). Die fehlende Expression der von den Antikörpern detektierten Proteine bei der Doppeldeletionsmutante konnte so verifiziert werden.

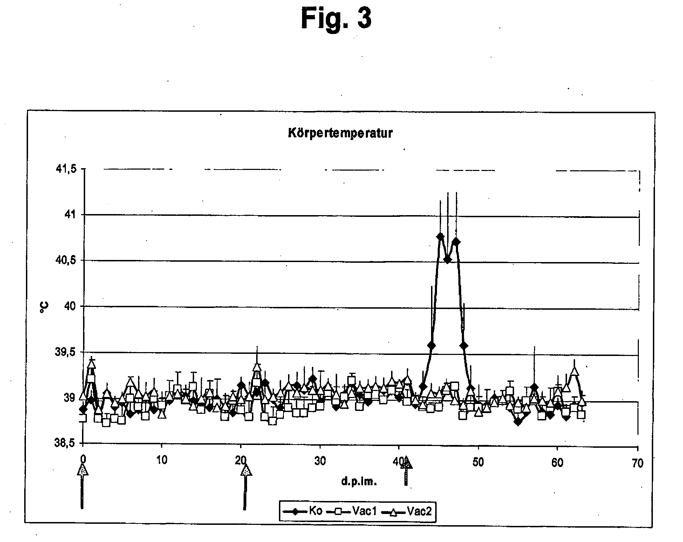

Figur 3 zeigt den Körpertemperaturverlauf der Rinder im Tierversuch. Dargestellt sind die Mittelwerte der Tiergruppen Vac1, Vac2 und der Kontrollgruppe Ko. Balken über den Messwerten kennzeichnen die Standardabweichung. Der kurze Pfeil kennzeichnet den Tag der Belastungsinfektion, während die langen Pfeile die Immunisierungstage markieren. Vac1 bezeichnet die Tiergruppe, die die Lebendimpfstoffzusammensetzung mit BHV-1ΔgEΔTK und Adjuvans Polygen^{™} erhalten hat. Vac2 steht für die Mittelwerte der Tiere, die allein das Impfvirus BHV-1ΔgEΔTK zur Immunisierung appliziert bekommen hatten.
   Mit Ko wird die Kontrolltiergruppe abgekürzt.

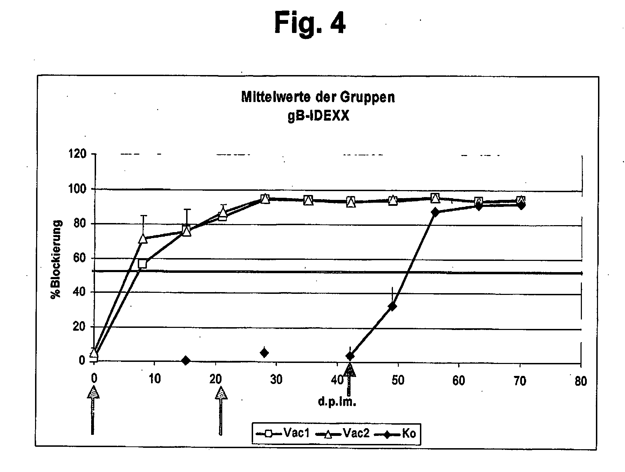

Figur 4 zeigt Ergebnisse der im Tierversuch untersuchten Tiergruppen im gB-IDEXX-ELISA: Das arithmetische Mittel der prozentualen Blockierung durch die Testseren der einzelnen Tiergruppen wird hier in Abhängigkeit von dem Zeitpunkt nach der Erstimmunisierung aufgetragen. Die waagrechte Linie stellt den Cut-Off Wert des Testes dar. Striche über den Messwerten zeigen jeweils die Standardabweichungen. Der kurze Pfeil kennzeichnet den Tag der Belastungsinfektion, während die langen Pfeile die Immunisierungstage markieren. Vac1 bezeichnet die Tiergruppe, die die Lebendimpfstoffzusammensetzung mit BHV-1ΔgEΔTK und Adjuvans Polygen^{™} erhalten hat. Vac2 steht für die Mittelwerte der Tiere, die allein das Impfvirus BHV-1ΔgEΔTK zur Immunisierung appliziert bekommen hatten. Mit Ko wird die Kontrolltiergruppe abgekürzt.

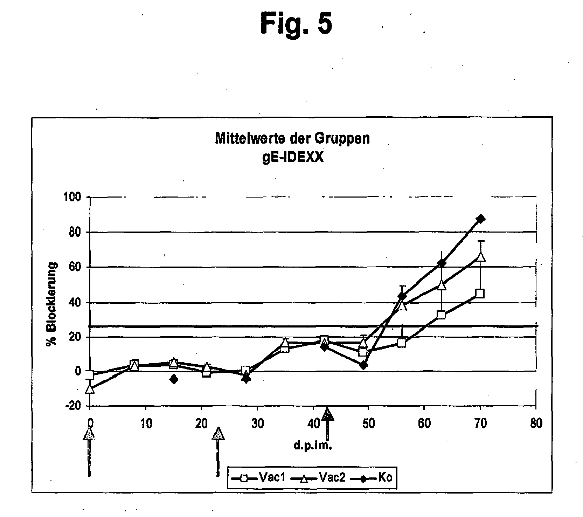

Figur 5 zeigt die Ergebnisse der im Tierversuch untersuchten Tiergruppen im gE-IDEXX ELISA-(Markertest): Die arithmetischen Mittelwerte jeder Tiergruppe nach Erstimmunisierung sind dargestellt. Mit der waagrechten Linie wird der Cut-Off-Wert für diesen Test angegeben. Die Standardabweichung der Mittelwerte wird durch Striche über den Messwerten dargestellt. Die langen Pfeile markieren die Immunisierung an Tag 0 und die Boosterung an Tag 21. Der kurze Pfeil kennzeichnet den Tag der Belastungsinfektion. Abkürzungen wie in Fig. 4.

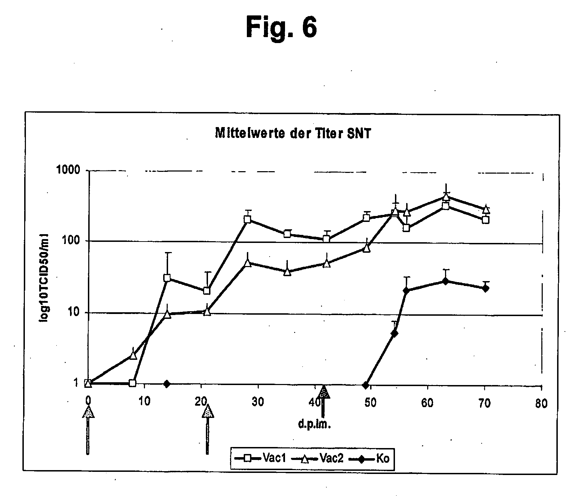

Figur 6 zeigt die Ergebnisse der im Tierversuch untersuchten Tiergruppen im Serumneutralisationstest (SNT). Dargestellt sind die arithmetischen Mittelwerte der SNT-Titer der Tiergruppen. Striche über den Messwerten symbolisieren die Standardabweichung. Der Tag, an dem die Belastungsinfektion erfolgte, ist mit einem kurzen Pfeil gekennzeichnet. Die langen Pfeile markieren die Tage, an denen die Tiere immunisiert wurden. Abkürzungen wie in Fig. 4.

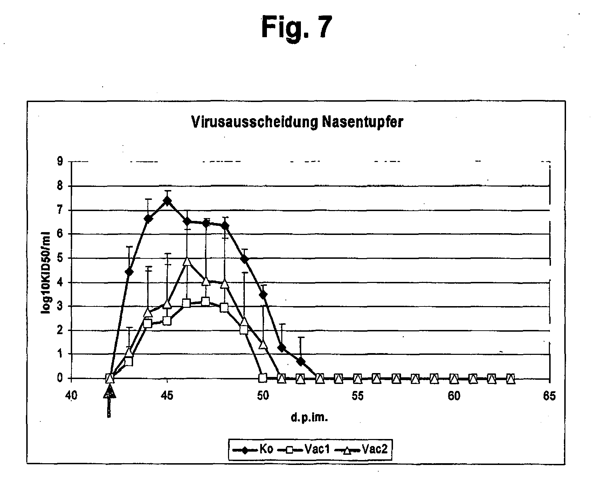

Figur 7 zeigt die Ergebnisse der Ermittlung der Virusausscheidung nach Belastungsinfektion. Dargestellt sind die durch Titration auf permissiven MDBK-Zellen erreichten Virustiter aus den täglich bis Tag 63 nach Immunisierung entnommenen Nasentupferproben. Der Tag der Belastungsinfektion ist durch einen Pfeil markiert. Standardabweichungen der Mittelwerte werden durch Striche über den Messwerten visualisiert. Abkürzungen wie in Fig. 4.

### Beispiel

### 1. Herstellung der gE/TK-deletierten BHV-1-Mutanten

Das gE/TK-deletierte BHV-1 wurde über das BAC-Mutagenesesystem (*Bacterial Artificial Chromosome*) ausgehend von dem BHV-1-Stamm "Schönböken" generiert. Dabei wurde das Virusgenom nach Insertion der BAC-Sequenz in Bakterien verbracht. Dadurch können alle weiteren Manipulationen des viralen Genoms mit Hilfe der etablierten Mutagenesetechniken, die von Bakterien bekannt sind, durchgeführt werden. Zusätzlich entfällt die Selektion von viralen Subpopulationen in Zellkultur, welche häufig zu weiteren undefinierten Veränderungen im viralen Genom führte.

Mit Hilfe der BAC-Mutagenese auf Basis des EL250-Systems wurde ein Virus generiert, das sowohl im gE-Genort aus auch im Genort UL23, der für das Enzym Thymidinkinase des BHV-1-Virus kodiert, partiell deletiert ist. Dazu wurde das Genom des BHV-1 pBHV-1ΔgE (Trapp S, Osterrieder N, Keil GM, Beer M; J. Gen. Virol. 2003;84(Pt 2):301-06) in elektrokompetente *E*. *coli* des Typs EL 250 (Lee EC, Yu D, Martinez de Velasco J, Tessarollo L, Swing DA, Court DL, Jenkins NA, Copeland NG ; Genomics. 2001;73(1):56-65) elektroporiert. Hierbei werden durch einen defekten Prophagen Funktionen vermittelt, die zur Protektion und Rekombination von transformierter DNA beitragen. Mittels PCR wurde eine lineare DNA generiert, die als Rekombinationspartner in EL250 BAC tragenden Zellen elektropororiert wurde. Dieses lineare DNA-Fragment verfügt über BHV-1-homologe Sequenzen, anhand derer die homologe Rekombination in das Genom des pBHV-1ΔgE erfolgt. Das Kanamycinresistenzgen des Plasmids pKD13 wurde als Selektionsmarker innerhalb der FRT-Sites verwendet.

**Tabelle 1: Für die TK-Deletion verwendete Primer**

| Den Positionsangaben liegen online verfügbare BHV-1 Sequenzdaten zugrunde (*NCBI Sequence NC_001847*)*.* Unterstrichene Buchstaben bezeichnen pKD13-spezifische Sequenzen, die für das Kanamycinresistenzgen kodieren. BHV-1 Sequenzen sind durch fett- und kursivgedruckte Buchstaben gekennzeichnet. | | |
|---|---|---|
| **Position** | **Strang** | **Sequenz (5'→ 3')** |
| 63682 | positiv | CTCTGCTACCCCTTCGCCCGCTACTGCCTC CGCGAGATCAACGCGGAAGATAGCGATTG TGTAGGCTGGAGC |
| 63975 | negativ | GCCATTTTGCATTCATCACAACTGGGGTCT GCGAGCGCGGCCAATGCATTTCGACCTGC AGTTCGAAGTTC |

Durch Aktivierung der Arabinose induzierbaren flpe-Rekombinase wurde das inserierte Kanamycinresistenzgen schließlich bis auf eine sogenannte FRT-Site von 35 bp aus dem Genom wieder entfernt. In Fig. 1 sind die Schritte der Mutagenisierung des pBHV-1DgE in *E.coli* schematisch dargestellt. Der genomische Aufbau des Wildtypvirus und die Restriktionsfragmentkarte desselben wird in Fig. 1A) verdeutlicht. Das Genumfeld der zu deletierenden Thymidinkinase ist in Fig. 1B) herausvergrößert. Fig. 1C) stellt die Insertion des Kanamycinresistenzgens dar, während in Fig. 1D) der Genomaufbau nach Elimination des Resistenzgens gegen das Antibiotikum wiedergegeben ist.

Der ORF der Thymidinkinase (63260..64339) konnte nicht komplett aus dem Genom entfernt werden, da ein wichtiger Promotor für benachbarte, essentielle Gene innerhalb dieses ORF gelegen ist. Aus diesem Grund wurden 293 Basenpaare (bp) von 63683 - 63975 deletiert. Um möglichst wenige Fremdsequenzen innerhalb des Virusgenoms mitzuführen, wurde die BAC-Sequenz innerhalb des gE-Locus entfernt. Dazu wurde eine Kotransfektion mit der DNA des doppelt deletierten BHV-1 und der Plasmid-DNA pTZ18RflanksgE durchgeführt. Die Plasmid-DNA pTZ18RflanksgE beinhaltet dabei zwei 1,4 kbp große BHV-1-homologe Sequenzen, die den gE-ORF bis auf einen Bereich von 281 bp (bp121694- bp121975) darstellen. Nach der Kotransfektion in Zellen des Typs MDBK entwickelten sich zwei Viruspopulationen: eine entsprach der transfizierten DNA, beinhaltete also noch die BAC-Sequenz und ließ sich durch die Expression von EGFP (*enhanced green fluorescent protein*) identifizieren. Die zweite Viruspopulation exprimierte dagegen kein EGFP, hatte also die BAC-Sequenz durch homologe Rekombination mit den Sequenzen der Plasmid-DNA pTZ18RflanksgE verloren. Diese Viruspopulation wurde durch Subpassagen unter semisolidem Medium weiter aufgereinigt.

### 2. Charakterisierung des Genoms der gE/TK-deletierten BNV-1-Mutante

### 2.1 Sequenzierung

Um das Virus auf genomischer Ebene zu charakterisieren, wurden die veränderten Genabschnitte sequenziert. Dreimal wurde unabhängig voneinander virale DNA gewonnen, die der Sequenzierung zugeführt wurde. Der Abgleich der gewonnenen Sequenzen erfolgte mit Hilfe der durch NCBI zur Verfügung gestellten Programme und Datenbanken.

Für den Genort der TK ergaben sich folgende Sequenzunterschiede im Vergleich zu den veröffentlichten Wildtyp-Sequenenzen:

An Position 63712 liegt beim Wildtyp ein C, während BHV-1ΔgEΔTK ein A aufweist. Die Aminosäuresequenz bleibt erhalten, da das so erhaltene Codon für dieselbe Aminosäure kodiert.

Bis 63730 ergibt sich keine weitere Veränderung und ab 63773 liegen ebenfalls keine Veränderungen vor. Zwischen 63730 und 63773 liegen 73 Basen, die nicht aus dem Virusgenom stammen. Ein Abgleich mit der Sequenz des Plasmids pKD13 (s.o.) ergab, dass einerseits die "natural FRT-site" und überlappend die Sequenz "distal 35 nt of natural FRT-site" im viralen Genom verblieben sind. Es handelt sich um unwesentlich mehr als die berechnete FRT-site, diese Sequenzen haben innerhalb des Genoms keine Funktion. Effektiv deletiert sind demnach 43 Basen des TK-Leserahmens.

Der Leserahmen des gE wies folgende Veränderungen verglichen mit den Wildtyp-Sequenzen auf:

An Position 121653 befindet sich G an Stelle von T und an Position 121654 A an Stelle von C. An Position 121657 fehlt das Nukleotid G, welches im Wildtypgenom vorkommt. Diese Änderungen der Basensequenz liegen in einer Region des BHV-1 Genoms, von der bis heute keine Funktion bekannt ist. Es wird angenommen, dass es sich um eine nicht kodierende Region handelt. An Position 121695 folgt die Schnittstelle der Restriktionsendonuklease PacI, die als Differenzierungsmerkmal zwischen Wildtypvirusgenom und BHV-1ΔgEΔTK dient (Restriktionsenzymverdau aufgereiniger Virus-DNA und Analyse im Gel). Nach dieser Schnittstelle folgen Sequenzen wie bei dem Wildtypvirus ab Position 121976. Damit weist BHV-1ΔgEΔTK eine Deletion des gE-Leserahmens von 121694 bis 121975 auf.

Die Sequenzanalysen zeigen, dass alle Deletionen vollständig sind und nur minimale, in der normalen Schwankungsbreite vorkommende Sequenzänderungen zu beobachten sind.

### 2.2 Western Blot Analyse

Die Überprüfung der Proteinexpression erfolgte über Immunoblot-Analysen von elektrophoretisch aufgetrennten Zelllysaten der untersuchten Viren. Vergleichend wurden das Wildtypvirus BHV-1 Schönböken, das einfach gE-deletierte Virus und das doppelt deletierte Virus BHV-1ΔgEΔTK überprüft. Dazu wurden MDBK-Zellen mit den entsprechenden Viren infiziert und nach 16-stündiger Inkubation wurden über einen Lysis-Schritt mit dem Detergenz Triton X-100 Zelllysate gewonnen. Mit BHV-1-spezifischen Antikörpern wurde die Expression von einerseits gE und andererseits TK getestet. Erwartungsgemäß detektierte das polyklonale Kaninchenserum pAK-TK1 eine Proteinbande von 37 kDa nur im Proteinmuster des Wildtypvirus BHV-1 Schönböken und in dem einfach gE-deletierten Virus (siehe Fig. 2A). Der monoklonale Antikörper mAk 2-1 reagierte spezifisch mit einer 94 kDa großen Proteinbande in Lysaten des Elternvirus, während eine solche Reaktion bei dem deletierten Virus unterblieb (Fig. 2B). Damit reagierte das doppelt deletierte Virus auf Proteinebene wie es korrekte Deletionen erwarten lassen würden. Die Immundetektion von gE und TK erfolgte an denselben Proben auf derselben Membran. Dies wurde durch einen als "Stripping" bekannten Schritt möglich, der der Behandlung mit dem Antikörper - dessen Antigen als zweites detektiert wurde - vorausgegangen war.

Insgesamt konnte mit Hilfe der Western-Blot-Analyse die Deletion der Proteine gE und TK ebenfalls bestätigt werden.

### 2.3 Southern Blot Analyse

Zur Überprüfung der Deletion des Glykoprotein-E-Leserahmens und der partiellen Deletion des Leserahmens der Thymidinkinase wurde ein Southern Blot angefertigt. Dabei wurde der Genotyp des mutierten Virus BHV-1ΔgEΔTK mit dem des Ausgangsvirus BHV-1 Stamm Schönböken verglichen. Die DNA der entsprechenden Viren wurde isoliert und mit Restriktionsenzym *Hind III* gespalten. Die Fragmente wurden in einem 0,7 % Agarosegel aufgetrennt.

Im Unterschied zum Ausgangsvirus wies die Deletionsmutante BHV-1ΔgEΔTK kein DNA-Fragment von 7,2 kbp auf. Durch die Insertion der BAC-Sequenz ist dieses Fragment auf 15,8 kbp vergrößert worden. Infolge der partiellen Deletion des TK-ORF verringerte sich die Größe des A-Fragments um 293 bp.

### 2.4 Konfokale Laserskanmikroskopie - Analyse der subzellulären Lokalisation von gE und TK

MDBK-Zellen wurden mit dem Wildtyp-Virus Schönböken und mit der Doppeldeletionsmutante BHV-1ΔgEΔTK infiziert. Die Oberfläche der unfixierten Zellen wurde jeweils gegen gE gefärbt. Als Sekundärantikörper wurde anti-Maus Alexa^{®}Fluor^{®}647 eingesetzt, dessen Fluoreszenz blau wiedergegeben wird. Die Plasmamembran wurde mit Vybrant^{™} Cell-Labeling Solutions DiI gefärbt. Diese hochgradig lipophile Substanz lässt sich durch Licht der Wellenlänge 549 nm anregen und die Fluoreszenz dieses Membranmarkers ist rot. Nach Fixation der Zellen durch 80 % Aceton wurden auch intrazelluläre Antigene durch entsprechende Antikörper detektierbar. Demzufolge wurde der polyklonale Antikörper zur Detektion von TK eingesetzt. Mit Anti-Kaninchen Alexa^{®}Fluor^{®}488 wurde der TK-Antikörper grün fluoreszierend markiert. Mit diesen 3 Farbspektren lassen sich die subzellulären Lokalisationen der entsprechenden Strukturen bzw. Proteine sichtbar machen. Bei wildtypinfizierten Zellen gelingt die Oberflächenfärbung gegen das gE. Diese Färbung verdeutlicht eine Kolokalisation mit der Plasmamembran der Zelle. Dagegen liefert die Markierung von TK ein punktförmiges Verteilungsmuster, das zudem vorrangig intrazytoplasmatisch auftritt. Zellkulturen, die mit dem doppelt deletierten Virus BHV-1ΔgEΔTK infiziert worden waren, reagierten ebenso wie die nicht infizierten Kontrollzellen allein mit der Markierungssubstanz für Biomembranen. Damit wurde die Abwesenheit der Proteine gE und TK nochmals bestätigt.

### 3. In-vitro-Charakterisierung der gE/TK-deletierten BHV-1-Mutante

### 3.1 Ein-Schritt-Wachstumskinetik

Zur Untersuchung des von Replikationseigenschaften des generierten BHV-1ΔgEΔTK wurden Ein-Schritt-Wachstumskinetiken durchgeführt. Hierzu wurden MDBK-Zellen eines Wells einer 24-Well-Zellkulturschale jeweils mit BHV-1-Schönböken (Wildtyp), BHV-1ΔgE und BHV-1ΔgEΔTK mit einer MOI (*Multiplicity of Infection*) von 1 (etwa 300.000 PFU) infiziert und eine Stunde lang unter Standardbedingungen inkubiert. Anschließend erfolgten zwei Waschschritte des infizierten Zellrasens mit PBS-Puffer (*Phosphate Buffered Saline,* pH 7,4: 137 mM NaCl; 2,7 mM KCl; 10 mM Na₂HPO₄; 2 mM KH₂PO₄). In der Folge wurde der Zellrasen für 2 min mit eiskaltem CBS-Puffer (*Citrate Buffered Saline*:40 mM Citronensäure, 10 mM KCl; 135 mM NaCl mit NaOH auf pH 3,0 eingestellt) bedeckt, um oberflächlich adsorbierende Viruspartikel zu inaktivieren. Erneut wurden die Zellen zweimal mit PBS-Puffer gewaschen und anschließend mit 1 ml Medium (DMEM (*Dulbecco's Modified Eagles's Medium*) + 10 % FKS (fötales Kälberserum)), welches auf 37 °C vorgewärmt wurde, versehen. Zur Bestimmung der Infektiosität im Zellkulturüberstand wurde nach 0 Std., 4 Std., 8 Std., 12 Std., 16 Std., 24 Std., 48 Std. und 72 Std. das Medium gewonnen und in ein 1,5 ml Reaktionsgefäß überführt. Zur Bestimmung der zellgebundenen Infektiosität wurde der Zellrasen, wie oben beschrieben, mit CBS-Puffer (pH 3,0) bedeckt und mit PBS-Puffer gewaschen. Anschließend wurden die Zellen mit einer Pipettenspitze von der Kulturschale abgeschabt, in 1 ml Medium (s.o.) aufgenommen und in ein 1,5 ml Reaktionsgefäß überführt. Die beiden Proben jedes Zeitwerts wurden bis zur Bestimmung des Virustiters bei -70 °C gelagert.

Es konnten keine deutlichen Wachstumsunterschiede zwischen dem Wildtyp und den deletierten Virusmutanten festgestellt werden. Somit hat die Deletion des Glycoproteins E in Kombination mit der Deletion der Thymidinkinase auf die hier quantifizierte BHV-1-Replikation keinen messbaren Einfluss.

### 3.2 Plaquegrößenbestimmung

Kulturschalen mit konfluenten MDBK-Zellen wurden mit 100 PFU (*Plaque Forming Units* / plaquebildende Einheiten) pro Well einer 6-Well-Zellkulturschale infiziert, nach einer Stunde mit semisolidem Methcel-Medium überschichtet, 48 Std. unter Standardbedingungen inkubiert und nach Kristallviolettfärbung ausgewertet. Die Vermessung der Plaques erfolgte mittels des Bildverarbeitungsprogramms analySIS^{®} (Fa. Softimaging System GmbH, Münster). Dabei wurden 150 Plaques pro Virus vermessen.

Der Mittelwert der Plaquegröße des Wildtypvirus wurde als Vergleichswert auf 100 % gesetzt. Das einfach gE-deletierte Virus erreichte 47 % der Vergleichsgröße während die Plaquegrößen des gE/TK-doppelt deletierten Virus bei etwa 37 % der Plaquegröße der Wildtypviren lagen.

Insgesamt zeigte die *in-vitro*-Charakterisierung von BHV-1ΔgEΔTK für die Wachstumskinetiken keine relevanten Unterscheide zum gE-einzeldeletierten Virus. Auch der Vergleich der Plaquegrößen zeigte kaum Unterscheide zwischen dem doppelt deletierten BHV-1ΔgEΔTK und dem einzeldeletierten BHV-1ΔgE. Das Virus BHV-1ΔgEΔTK verhielt sich insgesamt *in vitro* nahezu wie ein einzel-gE-deletiertes BHV-1 und ist somit voll replikationskompetent. So können in Zellkultur einfach hohe Virustiter erreicht werden und trotz doppelter Attenuierung (gE und TK) sind ausreichende Immunantworten zu erwarten.

### 4. Überprüfung von Adjuvantien auf Virustoxizität

Es wurden zwei Adjuvantien in einem zeit- und konzentrationsabhängigen Test hinsichtlich ihrer toxischen Wirkung auf die Virusmutante BHV-1ΔgEΔTK in Zellkultur überprüft. Dazu wurden Zellkulturen mit der Virusmutante beimpft und die Adjuvantien in Konzentration nach Herstellerangaben der virusbeimpften Zellkultur zugesetzt. Die Untersuchung auf virushemmende Wirkung der Adjuvantien erfolgte anhand der Auszählung der Virusplaques in Zellkultur.

Bei dem ersten geprüften Adjuvans Quil A, ein teilweise aufgereinigter Saponin-Extrakt aus *Quillaja saponaria,* wurde eine deutliche Virushemmung festgestellt. Das zweite geprüfte Adjuvans Polygen^{™} ist ein Copolmer mit niedrigem Molekulargewicht. Polygen^{™} schädigte das Virus nicht, da vergleichbar hohe Zellkulturtiter wie mit Wildtypvirus erhalten werden konnten.

Anschließend wurde Polygen^{™} im Tierversuch auf seine Wirkung im Tier untersucht. Es zeigte sich, dass Polygen^{™} an der Injektionsstelle (intramuskulär) keine Reaktionen hinterließ und keine Erhöhungen der Körpertemperatur im Tier induzierte. Darüber hinaus induzierte Polygen^{™} im Vergleich mit Freudschen Adjuvans nach Applikation mit dem immunisierenden Agens höhere Antikörpertiter im Tier.

Insgesamt erwies sich Polygen^{™} somit als geeignet für eine kombinierte Applikation von Lebendvirus mit Adjuvans.

### 5 Überprüfung der Eignung der gE/TK-deletierten BHV-1-Mutante mit und ohne Adjuvans als Lebendianpfstoff im Tierversuch

Zwölf Fleckviehrinder im Alter von neun Monaten wurden im Tierversuch zur Überprüfung der Eignung der gE/TK-deletierten BHV-1-Mutante als Lebendimpfstoff eingesetzt. In der serologischen Überprüfung der Tiere vor versuchbeginn konnten keine BHV-1-Antikörper nachgewiesen werden. Vier der Tiere wurden als Kontrolltiere (Ko) allein einer Belastungsinfektion ausgesetzt. Vier Tiere (Vakzinegruppe Vacl) wurden zweimalig im Abstand von 21 Tagen intramuskulär mit dem Lebendvirus BHV-1ΔgEΔTK in Kombination mit dem Adjuvans Polygen^{™} (12% v/v) immunisiert. Weitere vier Tiere (Vakzinegruppe Vac2) wurden mit derselben Virusdosis ohne Adjuvans immunisiert. 21 Tage nach der zweiten Immunisierung wurde intranasal eine Belastungsinfektion mit dem virulenten BHV-1-Wildtypstamm 2204 vorgenommen, in deren Folge den Tieren täglich Nasentupfer und wöchentlich Blut entnommen wurden.

Die Vakzinierungsdosis betrug 1x10⁷ KID₅₀ pro Tier. Die vakzine wurde in die Halsmuskulatur verimpft. Die Dosis der Belastungsinfektion betrug 1x10⁷ KID₅₀ pro Nasenloch jedes Tieres. Die Tiergruppen wurden im Isolierstall unter gleichen Bedingungen gehalten.

### 5.1 Temperatur

Über den gesamten Versuchszeitraum wurden die Tiere rektal thermometriert. Die Mittelwerte der Tiergruppen sind in Fig. 3 dargestellt. Die Schwankungen zwischen den Tiergruppen sind vor der Belastungsinfektion geringfügig. Nach der Belastungsinfektion entwickelten die Kontrolltiere Fieber über fünf Tage, während die vakzinierten Tiergruppen Vac1 und Vac2 kein Fieber zeigten.

### 5.2 Untersuchung von Serumproben auf gB- und gE-Antikörper

Die aus dem Tierversuch gewonnen Serumproben wurden mittels ELISA-Technik auf gE- und gB-Antiköper untersucht.

Die Abklärung der BHV-1-Situation erfolgte gemäß Tabelle 1:

**Tabelle 2**

| gB-ELISA | gE-ELISA | Beurteilung |
|---|---|---|
| Positiv | Positiv | BHV-1 Feldvirusinfektion oder geimpft mit konventionellem Impfstoff ohne Marker |
| Positiv | Negativ | Mit gE-deletiertem Markerimpfstoff geimpftes Tier |
| Negativ | Negativ | BHV-1 freies Tier |
| Negativ | Positiv | Ergebnis des gE-ELISA ist falsch positiv |

### gB-ELISA

Bei der serologischen Untersuchung auf gB-Antikörper wurde der kommerzielle Testkit der Firma IDEXX verwendet. Die Testdurchführung erfolgte nach den Herstellerangaben. Es handelt sich hierbei um ein kompetetives Testsystem, d.h. sind in der unbekannten Probe gB-Antikörper vorhanden, wird ein vom Test vorgegebenes Antigen blockiert und kann von dem Testsystem nicht mehr detektiert werden. Eine Probe gilt demnach als positiv, wenn die Blockierung durch die Probe in % ≥ 55 % beträgt. Liegt der Blockierungwert zwischen 45 % und 55%, gilt die Probe als fraglich. Als negativ werden Proben bewertet, deren Blockierungswert < 45 % liegt.

Im gB-ELISA wurden in den Serumproben der Tiere der Vakzinegruppen Vac1 und Vac2 bereits eine Woche nach der Immunisierung Meßwerte ermittelt, die zu einer positiven Bewertung des Testes führten (Fig. 4). Die Kontrolltiere wurden 14 Tage nach der Belastungsinfektion von dem Testsystem positiv bewertet.

### gE-ELISA

Zur Ermittlung des gE-Antiköperstatus einer Serumprobe wurde der gE-ELISA der Firma IDEXX eingesetzt. Die Testdurchführung erfolgte den Angaben des Herstellers entsprechend. Das Testprinzip dieses ELISA entspricht dem des gB-ELISA. Die Auswerung des Tests wird mittels Spektralphotometrie bei einer Wellenlänge von 650 nm durchgeführt. Die Bewertung erfolgt gemäß Herstellerangaben direkt anhand der gemessenen Absorptionswerte, die in Blockierungs-% umgerechnet wurden.
Der in der Markerdiagnostik eingesetzte gE-ELISA reagierte mit konstant negativen Messwerten bei der Untersuchung der Serumproben aller vakzinierten Tiere vor der Belastungsinfektion. Zwei Wochen nach der Wildtypvirus-Infektion wurden die höchsten Messwerte für die Kontrolltiere im gE-ELISA ermittelt. Die gE-ELISA-Werte der Tiergruppe Vac1, die mit der adjuvierten Lebendimpfstoffzusammensetzung immunisiert wurden, überschritten den Grenzwert des Testes hin zu einer positiven Bewertung hingegen erst drei Wochen nach der Belastungsinfektion (Fig. 5).

### 5.3 Serumneutralsationstest (SNT)

Mit Hilfe des Serumneutralisationstests wurde der Titer virusneutralisierender Antikörper in den Serumproben bestimmt. Zur Vorbereitung der Seren wurden diese 30 min bei 56 °C im Wasserbad inaktiviert. Anschließend wurden die Serumproben ebenso wie Referenzseren (Positiv, Negativserum) in log2-Verdünnungsschritten jeweils drei Proben pro Verdünnungsschritt (50 µl/Well) auf eine 96 Well-Platte gebracht. Das Testvirus wurde in einer Konzentration von 10² KID₅₀/50 µl verwendet. 50 µl dieses Testvirus wurden den Serumverdünnungen zugesetzt und nachfolgend für 24 Std. bei 37 °C und 5 % CO₂ inkubiert. Zur weiteren Validierung des Testsystems erfolgte jeweils eine Rücktitration des eingesetzen Testvirus. Im Anschluß an die Inkubation wurden 100 µl einer MDBK-Zellsuspension (30000 Zellen/ 100 µl) zu allen Vertiefungen gegeben. Die Bewertung des Tests wurde nach viertägiger Inkubation bei 37 °C und 5 % CO₂ durchgeführt. Dabei wurde der Neutralisationstiter berechnet anhand der Formel (-log2) = a/b + c, wobei a der Anzahl der Vertiefungen ohne Virusvermehrung entspricht, b die Anzahl der Vertiefungen pro Verdünnungsstufe wiedergibt und c -log2 einer eventuellen Vorverdünnung der eingesetzen Testseren entspricht.

Die Mittelwerte der im SNT gemessenen, neutralisierenden Antikörper der beiden Vakzinegruppen Vac1 und Vac2 unterschieden sich insofern, dass die Tiergruppe Vac1, welche die Lebendimpfstoffzusammensetzung mit Adjuvans Polygon^{™} erhalten hatte, fast durchgehend vor der Belastungsinfektion höhere BHV-1-Antikörpertiter aufwies (Fig. 6). Nach der Belastungsinfektion glichen sich die Titer der Tiere der beiden Gruppen an. Die Kontrolltiere entwickelten erst zu diesem Zeitpunkt neutralisierende Antikörper. Die Kurven der Messwerte, die für die vakzinierten Tiere ermittelt wurden, zeigten zudem deutlich die Stimulation der Antikörperantwort durch die Boosterimmunisierung wie auch die Belastungsinfektion.

### 5.4 Quantifizierung der nasalen Virusexkretion

Nasentupferproben wurden nach der Erstimmunisierung 10 Tage lang täglich und nach der Belastungsinfektion täglich entnommen. Mittels Virustitration auf permissiven MDBK-Zellen wurde die Exkretion von infektiösem BHV-1 quantifiziert. MDBK-Zellen wurden zur Durchführung von Virustitrationen in 96-Loch-Mikrotiterplatten genutzt (Mayr, A., Bachmann, P.A., Bibrack, B., Wittmann, G. (1974) Virologische Arbeitsmethoden Band I, Gustav Fischer Verlag, Stuttgart). Hierzu wurden die infektiösen Virusaliquots in log10-Verdünnungsschritten verdünnt und auf die Zellen gegeben und nachfolgend für drei Tage unter Standardbedingungen inkubiert. Anschließend wurden die Zellen mikroskopisch auf die Ausbildung eines zpE (zytopathogener Effekt) hin untersucht. Der Gehalt an infektiösen Viruspartikeln wurde als KID₅₀/ml (kulturinfektiöse Dosis 50% pro ml) nach der Methode von Spaerman und Kaerber (Mayr et al., 1974) berechnet.

Nach der Erstimmunisierung wurde in keiner der Nasentupferproben infektiöses Virus nachgewiesen.

Die nasale Virusausscheidung erreichte bei den Kontrolltieren den höchsten mittleren Titer mit 10^{7,375} KID₅₀/ml am dritten Tag nach der Belastungsinfektion und konnte bei dieser Tiergruppe im Mittel über elf Tage beobachtet werden. Die Tiergruppe Vac2, die alleine das Impfvirus zur Immunisierung erhalten hatte, schied über neun Tage nasal Virus aus, wobei die Virustiter mindestens um eine, maximal um vier log-Stufen reduziert waren. Die mittleren Messwerte der Tiergruppe Vac1, die mit Lebendvirus und Adjuvans Polygen^{™} immunisiert worden war, zeigte eine minimale Titerreduktion von zwei log10-Stufen und eine maximale von fünf log10-Stufen. Die Virusausscheidung war verkürzt und konnte im Mittel über acht Tage nachgewiesen werden (Fig. 7).

Das eingesetzte Lebendvirus erwies sich als vollkommen avirulent in beiden Tiergruppen. Die intramuskuläre Immunisierung führte außerdem zu keiner nachweisbaren Replikation des Impfvirus in der nasalen Mukosa. Da nach Immunisierung keinerlei Virusexkretion nachweisbar war, ist es wahrscheinlich, dass durch das Impfvirus keine Zellen latent infiziert wurden.

Nach der intranasalen Belastungsinfektion zeigten beide vakzinierten Tiergruppen deutlich verringerte Virustiter in den Proben der Nasentupfer und die Dauer der Virusausscheidung über die Nase war erheblich verkürzt verglichen mit den nicht vakzinierten Kontrolltieren. Die Tiergruppe Vac1 zeigte eine immunologisch günstigere Entwicklung hinsichtlich der Antikörpertiter und nasalen Virusexkretion, welche sich auf den zusätzlichen Einsatz des Adjuvans zurückführen lässt.

Die Tiere der Tiergruppe Vac1 entwickelten eine derart protektive Immunitätslage, dass die Belastungsinfektion frühestens drei Wochen nach Testinfektion durch den Markertest (gE-Antikörper-ELISA) detektiert werden konnte.

Dies spricht für einen besonders ausgeprägten Schutz vor einer Belastungsinfektion mit virulentem BHV-1.

## Patentansprüche

1. Lebendimpfstoffzusammensetzung, enthaltend eine gE- und TK deletierte BHV-1-Mutante in einem geeigneten Träger und ein nicht viruzides Adjuvans.

2. Lebendimpfstoffzusammensetzung nach Anspruch 1, bei der das nicht viruzide Adjuvans ein Polymer, insbesondere ein Copolymer oder Block-Copolymer ist.

3. Lebendimpfstoffzusammensetzung nach Anspruch 2, bei der das nicht viruzide Adjuvans Polygen^{™} ist.

4. Lebendimpfstoffzusammensetzung nach einem der vorstehenden Ansprüche, bei der der Anteil des nicht viruziden Adjuvans 5 bis 50 % (v/v) beträgt.

5. Lebendimpfstoffzusammensetzung nach Anspruch 4, bei der der Anteil des nicht viruziden Adjuvans 10 bis 15 % (v/v) beträgt.

6. Verwendung der Lebendimpfstoffzusammensetzung nach einem der Ansprüche 1 bis 5 zur Herstellung einer Arzneimittelformulierung zur Prophylaxe von BHV-1-Infektionen und BHV-1-vermittelten Erkrankungen.

7. Verfahren zur Herstellung einer gE- und TK-deletierten BHV-1-Mutante mit den Schritten
A. Transfizieren der DNA von BHV-1ΔgE und ein Rekombinationsplasmid mit einer BAC-Kassette in eukaryotische Zellen.
B. Insertion der BAC-Kassette in die DNA von BHV-1ΔgE zur Bildung von BHV-1ΔgE-BAC-DNA;
C. Elektroporieren von Plasmiden der BHV-1ΔgE-BAC-DNA in elektrokompetente *E*. *coli;*
D. Mutagenisierung des BHV-1ΔgE-BAC-Klons in *E*. *coli* durch homologe Rekombination zur Bildung von BHV-1ΔgEΔTK-BAC-DNA;
E. Selektieren der mutagenisierten BHV-1ΔgEΔTK-BAC-DNA;
F. Transfizieren der mutagenisierten BHV-1ΔgEΔTK-BAC-DNA in eukaryotische Zellen;
G. Generieren von gE- und TK-deletierten BHV-1-Nachkommen.

8. BHV-1-Mutante, erhältlich durch ein Verfahren nach Anspruch 7.
